# EUROPÄISCHE PATENTSCHRIFT

(11) **EP 4 347 137 B1**
(45) Veröffentlichungstag und Bekanntmachung des Hinweises auf die Patenterteilung: **09.07.2025**
(21) Anmeldenummer: 22730890.5
(22) Anmeldetag: 30.05.2022
(51) Int. Cl.: A61L 2/00, B04B 5/04, B04B 7/02, B04B 15/06, B04B 7/08

(54) **ZENTRIFUGE ZUM ROTIEREN EINES PROBENTRÄGERS**
CENTRIFUGE FOR ROTATING A SAMPLE CARRIER
CENTRIFUGEUSE POUR FAIRE TOURNER UN PORTE-ÉCHANTILLON

(30) Priorität: 31.05.2021 LU 102813
(43) Veröffentlichungstag der Anmeldung: 10.04.2024
(73) Patentinhaber: CYTENA GMBH, 79106 Freiburg (DE)
(72) Erfinder: RIBA, Julian, 79106 Freiburg (DE); RAUCH, David, 79106 Freiburg (DE); OPGENORTH, Roman, 79106 Freiburg (DE)
(74) Vertreter: Grabovac, Dalibor
(86) Internationale Anmeldenummer: PCT/EP2022/064564
(87) Internationale Veröffentlichungsnummer: WO 2022/253737

(56) Entgegenhaltungen:
- WO-A1-2018/234420
- WO-A1-2019/165478
- WO-A1-2021/061406
- CN-A- 106 890 732
- CN-A- 111 495 615

## Beschreibung

Die Erfindung betrifft eine Zentrifuge zum Rotieren eines Probenträgers. Darüber hinaus betrifft die Erfindung ein Verfahren beim Betreiben einer Zentrifuge.

Aus dem Stand der Technik sind Zentrifugen bekannt, in die Probenträger eingebracht werden können. Die Probenträger weisen wenigstens ein Behältnis auf, das zum Aufnehmen von einer flüssigen Probe dient. Die Zentrifuge weist einen Rotor mit einem Aufnahmeabschnitt auf, der den Probenträger aufnimmt. Der Rotor ist in einem Rotorraum der Zentrifuge angeordnet und dreht sich in diesem. Infolge der Drehung des Rotors wird ein Teil der flüssigen Probe aus dem Probenträger infolge der auf die flüssige Probe wirkenden Zentrifugalkraft ausgeschleudert. Somit verbleibt in dem Behältnis ein gewünschtes biologisches Partikel, das anschließend weiter prozessiert werden kann, insbesondere kann durch eine Dispensiervorrichtung Flüssigkeit in das Behältnis eingebracht werden, die anschließend erneut aus dem Behältnis ausgeschleudert werden kann. Ein den Rotorraum umschließendes Rotorgehäuse weist einen Auslass auf, durch den die ausgeschleuderte Flüssigkeit und/oder Partikel aus dem Rotorraum abgeführt werden. Die Zentrifuge kann beispielsweise eingesetzt werden, um Flüssigkeit und/oder biologische Partikel aus dem Behältnis zu entfernen, sodass lediglich gewünschte Zellen und/oder magnetische Beads, an denen DNA oder Proteine angelagert ist, in dem Behältnis verbleiben. Eine derartige Zentrifuge ist beispielsweise aus der WO 2015 0188 78 A1 bekannt.

Es kann jedoch vorkommen, dass zumindest ein Teil der ausgeschleuderten Probe nicht vollständig aus dem Rotorraum abgeführt werden kann. Da der aus der Zentrifuge nicht abgeführte Teil der flüssigen Probe beispielsweise Mikroorganismen oder DNA aufweisen kann, besteht ein Risiko, dass flüssige Proben die anschließend gewaschen werden, durch den im Rotorraum befindlichen Teil der flüssigen Probe, der nicht abgeführt wurde, kontaminiert werden.

CN 106 890 732 A weist eine Zentrifuge, die einen Rotor mit mehreren Aufnahmeabschnitten zum jeweiligen Aufnehmen von einem Behälter aufweist. Der Rotor ist in einem innerhalb der Zentrifuge vorgesehenen Hohlraum angeordnet. Die Zentrifuge weist eine Strahlungsquelle auf.

WO 2021/061 406 A1 offenbart eine Zentrifuge, die eine Antriebseinheit aufweist, auf die ein Container gesetzt wird. Die Zentrifuge weist außerdem eine Strahlungsquelle auf, die Strahlen mit einer Wellenlänge von 100 bis 450 Nanometer ausgibt.

CN 111 495 615 A offenbart eine Zentrifuge, die in einen Rotor aufweist, der in einem Hohlraum der Zentrifuge angeordnet ist.

WO 2019/165 478 A1 offenbart ein Rotorsystem mit einem Rotor. Das System weist eine elektromagnetische Strahlungsquelle auf, die an einer ersten Position des Rotors angeordnet ist.

WO 2018/ 234 420 A1 offenbart eine Zentrifuge zum Reinigen einer Reaktionsgefäßeinheit mit einem Rotor und einem Rotorraum, in welchem der Rotor drehbar gelagert ist. Der Rotorraum wird von einem Gehäuse begrenzt, das Rotors eine Ablaufrinne aufweist.

Die Aufgabe der Erfindung besteht daher darin, eine Zentrifuge bereitzustellen, bei der das Kontaminationsrisiko klein ist.

Die Aufgabe wird gelöst durch eine Zentrifuge zum Rotieren eines Probenträgers, der wenigstens ein Behältnis zum Aufnehmen einer flüssigen Probe aufweist, mit einem drehbaren Rotor, der wenigstens einen Aufnahmeabschnitt zum Aufnehmen des Probenträgers aufweist, und einem Rotorraum, in welchem der Rotor angeordnet, wobei die Zentrifuge wenigstens eine Strahlungsquelle zum Ausgeben einer Strahlung in den Rotorraum mit einer Wellenlänge von höchstens 350nm (Nanometer) aufweist, wobei der Rotorraum durch ein Rotorgehäuse begrenzt ist, dadurch gekennzeichnet, dass die Strahlungsquelle einen Strahler aufweist, der mittels eines Wärmeübertragungsabschnitts mit dem Rotorgehäuse wärmeleitend verbunden ist.

Eine weitere Aufgabe der Erfindung besteht darin, ein Verfahren bereitzustellen, mittels dem das Kontaminationsrisiko verkleinert wird.

Die Aufgabe wird gelöst durch ein Verfahren beim Betreiben einer erfindungsgemäßen Zentrifuge, wobei, insbesondere in einem Waschbetrieb, ein Probenträger, der wenigstens ein Behältnis zum Aufnehmen einer flüssigen Probe aufweist, mittels eines Rotors in einem Rotorraum gedreht wird, dadurch gekennzeichnet, dass, insbesondere in einem Dekontaminationsbetrieb, eine Strahlung mit einer Wellenlänge von höchstens 350nm (Nanometer) in den Rotorraum ausgegeben wird.

Der Vorteil der Erfindung besteht darin, dass erkannt wurde, dass das Risiko einer Kontamination verringert wird, wenn der Rotorraum mit einer Strahlung mit einer Wellenlänge von höchsten 350nm bestrahlt wird. Eine derartige Strahlung weist eine ausreichend hohe Energie auf, um die in der flüssigen Probe befindlichen biologischen Partikel abzutöten, die im Rahmen des Waschvorgangs aus dem Probenträger ausgeschleudert und nicht aus dem Rotorraum abgeführt wurden.

Besonders bevorzugt können biologische Partikel, insbesondere Mikroorganismen, abgetötet oder inaktiviert werden, wenn die Wellenlänge höchstens 315nm, vorzugsweise 280nm, beträgt. Eine untere Grenze des Strahlungsbereichs kann wenigstens 100nm, insbesondere 200nm, betragen. In einem solchen Fall kann die ausgegebene Strahlung eine Wellenlänge in einem Bereich von 100nm bis 350nm haben. Ganz besonders vorteilhaft, ist, wenn die ausgegebene Strahlung eine Wellenlänge von 254nm aufweist oder in einem Bereich zwischen 260nm bis 265nm liegt.

Der Probenträger weist ein oder mehrere Behältnisse auf und dient zum Aufnehmen von flüssiger Probe. Insbesondere kann die flüssige Probe in dem Behältnis oder den Behältnissen angeordnet sein. Dabei kann der Probenträger eine Mikrotiterplatte sein. Mikrotiterplatten können mit einer unterschiedlichen Anzahl an Behältnissen ausgebildet sein. So sind Mikrotiterplatten mit 6 bis 3456 Behältnissen bekannt, wobei üblicherweise Mikrotiterplatten mit 96, 384 oder 1536 Behältnissen verwendet werden. Dabei kann der Probenträger eine Aufnahmeeinrichtung aufweisen, in die die Mikrotiterplatte eingesetzt wird. Der Aufnahmeabschnitt des Rotors kann derart ausgebildet sein, dass eine Normale zu einer Behältnisöffnung senkrecht zu einer Rotorwelle steht. Dies bedeutet, dass sich eine Längsachse des Behältnisses des Probenträgers in eine Richtung erstreckt, die senkrecht zu einer Erstreckungsrichtung der Drehachse der Rotorwelle ist. Im Ergebnis verläuft eine Ebene, die eine Oberfläche des Aufnahmeabschnitts aufweist, auf die der Probenträger gesetzt wird, parallel zu der Drehachse des Rotors.

Die flüssige Probe kann eine Flüssigkeit und biologische Partikel aufweisen. Die biologischen Partikel können Mikroorganismen wie Bakterien, Archaean, Hefen, Pilze, und Viren oder Zellen, DNA, RNA oder Proteine sein. Die flüssige Probe kann ein einziges oder mehrere der zuvor genannten biologischen Partikel aufweisen.

Als Strahlungsquelle wird jeder Einrichtung verstanden, mittels der eine Strahlung mit einer Wellenlänge von höchstens 350nm erzeugt werden kann. Dies umfasst auch Einrichtungen mittels denen Strahlungen mit Wellenlängen erzeugt werden können, die nur einen Teilbereich des zuvor genannten Bereichs abdecken. Dies bedeutet, dass die Strahlungsquelle nicht den gesamten Strahlungsbereich, nämlich 100nm bis 350nm, abdecken muss.

Die Zentrifuge kann in einem Waschbetrieb betrieben werden. Als Waschbetrieb wird ein Betrieb der Zentrifuge verstanden, bei dem der Rotor mit einer Drehzahl gedreht wird, die ausreichend hoch ist, dass, insbesondere ein Teil, der flüssigen Probe infolge der wirkenden Zentrifugalkräfte aus dem Behältnis oder den Behältnissen ausgeschleudert wird. Es kann jedoch dafür gesorgt werden, dass bestimmte biologische Partikel entgegen der Zentrifugalkraft im Behältnis zurückgehalten werden. Nach Abschluss des Waschbetriebs verbleibt somit das gewünschte biologische Partikel in dem Behältnis.

Das Zurückhalten der biologischen Partikel kann beispielsweise über Zelladhäsion, magnetische Kräfte, kovalente chemische Binden oder ähnliches geschehen. So ist ein Waschbetrieb möglich, bei dem ein Behältnisboden mit einer bestimmten Beschichtung versehen ist, an dem die biologischen Partikel haften. Darüber hinaus ist ein Waschbetrieb möglich, bei dem magnetische Beads eingesetzt werden. Bei diesem Waschbetrieb werden Biomoleküle, wie DNA, RNA oder Proteine, zunächst an magnetische Beads gebunden und diese dann mittels eines Magnets, welcher unterhalb des Behältnisses in der Zentrifuge positioniert ist, am Behältnisboden gehalten. Es ist auch möglich, dass Zellen an magnetische Beads gebunden werden.

Außerdem ist ein Waschbetrieb bekannt, bei dem Proteine an den Behältnisboden gebunden werden, welcher zuvor mit einer bestimmten Reagenz versehen worden ist. Bei einem anderen Waschbetrieb werden Zellen, insbesondere Suspensionszellen, zunächst stark zentrifugiert, damit sich ein sogenanntes Pellet am Boden bildet. Das Pellet verbleibt dann im Behältnis, wenn ein Teil der flüssigen Probe herausgeschleudert wird.

Als Dekontaminationsbetrieb wird ein Betrieb verstanden, bei dem Strahlung ausgegeben wird, um die in dem Rotorraum befindlichen biologischen Partikel abzutöten oder zu zersetzen.

Im Dekontaminationsbetrieb wird ausgenutzt, dass durch Bestrahlen von DNA und RNA mit Strahlung einer bestimmten Wellenlänge erreicht wird, dass sie nicht weiter repliziert werden können oder mit der Zeit zerfallen. Werden Zellen Strahlung einer bestimmten Wellenlänge ausgesetzt, so beobachtet man häufig eine Strukturänderung der DNA, die auf die Bildung von Thymidin-Dimeren zurückzuführen ist. Zwei auf einem DNA-Strang benachbarte Thymidine werden dabei kovalent über einen Cyclobutan-Ring verbunden. Bei dem Mechanismus handelt es sich um eine photoinduzierte Cycloaddition. Durch die Aufnahme eines Lichtquants wird ein Thymidin-Molekül angeregt, und dadurch kann es nun zur Wechselwirkung mit einem Thymidin im Grundzustand kommen. Dabei wird in beiden Molekülen die Doppelbindung zwischen dem C5 und dem C6-Atom des Thymins aufgehoben. Gleichzeitig bilden sich zwischen den C5-Atomen und den C6-Atomen beider Moleküle Einzelbindungen aus. Die Folge einer solchen Thymidin-Dimerbildung ist eine Änderung in der räumlichen Gestalt der DNA. Proteine, die mit der DNA wechselwirken, können das entstandene Dimer nicht passieren. Deshalb kommen Transkription und Replikation an diesen Stellen zum Erliegen.

Im Ergebnis wird eine Zentrifuge bereitgestellt, die wahlweise in einem Waschbetrieb oder einem Dekontaminationsbetrieb betrieben werden kann. Darüber hinaus wird ein Verfahren zum Betreiben einer Zentrifuge bereitgestellt.

Bei einer besonderen Ausführung kann das Rotorgehäuse eine Schale aufweisen. Die Schale kann den Rotorraum in radialer und/oder tangentialer Richtung begrenzen. Die Schale kann mehrteilig ausgebildet sein. So kann die Schale eine obere Schale und eine untere Schale aufweisen, die wieder lösbar miteinander verbunden sein können. Die obere Schale ist auf der unteren Schale angeordnet. Dabei kann die obere und untere Schale derart ausgebildet sein, dass es den Rotorraum, insbesondere in radialer, axialer und tangentialer Richtung, begrenzt. Im Ergebnis kann ein kompaktes Rotorgehäuse bereitgestellt werden.

Ein besonders kompaktes Rotorgehäuse wird realisiert, wenn das Rotorgehäuse, insbesondere die obere Schale, eine zylinderförmige Gehäuseinnenfläche aufweist. Das Rotorgehäuse kann in einer Normalebene, die Normal zu einer nachstehend näher beschriebenen Rotorwelle steht und einen Teil der oberen und/oder unteren Schale umfasst, die zylinderförmige Gehäuseinnenfläche aufweisen. Dabei wird als Gehäuseinnenfläche die den Rotorraum unmittelbar begrenzende Fläche des Rotorgehäuses verstanden. Die zylinderförmige Gehäuseinnenfläche kann durch eine entsprechende Ausbildung der oberen Schale und/oder unteren Schale realisiert werden. Die zylinderförmige Gehäuseinnenfläche ermöglicht, dass der Rotorraum, in dem sich der Rotor dreht, möglichst kompakt ausgebildet ist, also der Abstand zwischen dem Rotor und dem Rotorgehäuse, insbesondere in radialer Richtung, klein ist.

Als "radiale", "tangentiale" und "axiale" Richtungsangabe wird jeweils eine Richtung verstanden, die sich auf eine Mittelachse des Rotorgehäuses bezieht. Die Mittelachse kann koaxial oder parallel zu einer Längsachse der Zentrifuge und/oder einer Mittelachse der Rotorwelle verlaufen. Die Mittelachse der Rotorwelle entspricht der Drehachse der Rotorwelle und somit des Rotors

Das Rotorgehäuse kann derart ausgebildet sein, dass es einen Austritt der Strahlung aus dem Rotorraum verhindert. Zum Verhindern eines Strahlungsaustritts kann das Rotorgehäuse eine vordere Wandung und eine hintere Wandung aufweisen. Die vordere und hintere Wandung begrenzen den Rotorraum, insbesondere in axialer Richtung. Der Rotorraum wird somit durch die obere Schale, die untere Schale, die vordere und hintere Wandung begrenzt. Die vordere Wandung entspricht der Wandung, die einer Zentrifugenaufnahme näher zugeordnet ist als die hintere Wandung. Durch die zuvor beschriebene Ausbildung des Rotorgehäuses kann auf einfache Weise verhindert werden, dass die Strahlung aus dem Rotorraum austritt und auf flüssige Proben trifft, die außerhalb des Rotorraums angeordnet sind. Außerdem wird verhindert, dass z.B. ein Nutzer der Zentrifuge mit der energiereichen Strahlung in Kontakt kommt.

Bei einer besonderen Ausführung kann die Zentrifuge die Rotorwelle aufweisen, die den Rotor antreibt. Die Rotorwelle kann an dem von dem Rotor abgewandten Ende mit einem Motor triebtechnisch verbunden sein. Dabei kann die Rotorwelle in der vorderen und hinteren Wanderung jeweils drehbar gelagert sein. Dabei kann die Rotorwelle durch die hintere Wandung hindurch verlaufen und in einen Elektronikraum der Zentrifuge eindringen, indem der Motor angeordnet ist. Die hintere Wandung trennt somit den Rotorraum der Zentrifuge von dem Elektronikraum, insbesondere verhindert die hintere Wandung, dass ausgeschleuderte flüssige Probe in den Elektronikraum eindringt. Dies ist vorteilhaft, weil in dem Elektronikraum zusätzlich zu dem Motor weitere elektrische oder elektronische Geräte, wie beispielsweise eine Steuervorrichtung, angeordnet sein können und somit ein Eintritt von flüssiger Probe in den Elektronikraum verhindert werden soll.

Eine Mittelachse der Rotorwelle kann parallel zu einer Standfläche der Zentrifuge verlaufen. Insbesondere kann eine Mittelachse der Rotorwelle koaxial oder parallel zu der Mittelachse des Rotorgehäuses verlaufen. Die Standfläche kann durch eine Unterseite der unteren Schale oder eine Unterseite von Standfüßen der Zentrifuge ausgebildet sein. Darüber hinaus kann die Mittelachse der Rotorwelle parallel zu einer Einbringrichtung oder Ausbringrichtung des Probenträgers in den Rotor verlaufen. Die Ausbringrichtung kann entgegengesetzt zu der Einbringrichtung gerichtet sein. Als Einbringrichtung wird die Richtung verstanden, entlang der der Probenträger bewegt wird, um den Probenträger von einer Zentrifugenaufnahme in den Aufnahmeabschnitt zu bewegen.

Die vordere Wandung kann einen Durchgang aufweisen, durch den der Probenträger in den Rotor, insbesondere einen Aufnahmeabschnitt, eingebracht werden kann. Somit kann der Probenträger auf einfache Weise in den Rotor eingebracht werden. Darüber hinaus kann der Probenträger durch denselben Durchgang aus dem Rotor ausgebracht werden. Die Zentrifuge kann eine Verschiebeeinrichtung aufweisen, mittels der der Probenträger in den Rotor eingebracht oder aus dem Rotor ausgebracht wird. Im Ergebnis kann, insbesondere nach einem Einbringen des Probenträgers in eine Zentrifugenaufnahme, der Probenträger mittels der Verschiebeeinrichtung automatisch in den Rotor eingebracht werden. Darüber hinaus kann der Probenträger mittels der Verschiebeeinrichtung automatisch aus dem Rotor in die Zentrifugenaufnahme ausgebracht werden.

Die Verschiebeeinrichtung kann zum Verschieben des Probenträgers mit dem Probenträger wieder löslich verbunden werden. Insbesondere kann die Verschiebeeinrichtung mit dem Probenträger magnetisch verbunden werden. Zum Verbinden des in der Zentrifugenaufnahme angeordneten Probenträgers erstreckt sich die Verschiebeeinrichtung durch den Rotor, insbesondere den Aufnahmeabschnitt, hindurch. Die Verschiebeeinrichtung zieht den Probenträger in den Aufnahmeabschnitt des Rotors. Darüber hinaus schiebt die Verschiebeeinrichtung den Probenträger in die Zentrifugenaufnahme zurück, nachdem der Waschbetrieb abgeschlossen ist. Dabei ist die Verschiebeeinrichtung während des Waschbetriebs und des Dekontaminationsbetriebs nicht im Rotorraum angeordnet.

Die Zentrifuge kann eine Klappe zum Verschließen des Durchgangs der vorderen Wandung aufweisen. Dabei kann der Durchgang durch die Klappe, insbesondere vollständig, verschlossen sein, wenn sich die Zentrifuge im Waschbetrieb befindet. Darüber hinaus kann die Klappe den Durchgang, insbesondere vollständig, verschließen, wenn sich die Zentrifuge im Dekontaminationsbetrieb befindet. Im Dekontaminationsbetrieb strahlt die Strahlungsquelle Strahlung aus, um die biologischen Partikel, insbesondere Mikroorganismen, die im Rotorraum verblieben sind, abzutöten.

Bei einer besonderen Ausführung kann das Rotorgehäuse die wenigstens eine Strahlungsquelle aufnehmen und/oder mit der wenigstens einen Strahlungsquelle, insbesondere fest verbunden sein. Als feste Verbindung wird eine Verbindung verstanden, bei der sich die beiden miteinander verbundenen Komponenten nicht relativ zueinander bewegen können. Dazu kann die wenigstens eine Strahlungsquelle am oder im Rotorgehäuse angeordnet sein. Insbesondere kann das Rotorgehäuse eine Aussparung aufweisen, in der die wenigstens eine Strahlungsquelle angeordnet ist und/oder in die sich die Strahlungsquelle wenigstens teilweise erstreckt. Vorteilhaft ist es, wenn die vordere Wandung und/oder die hintere Wandung eine Aussparung aufweisen, in der wenigstens eine Strahlungsquelle angeordnet ist und/oder in die sich die Strahlungsquelle wenigstens teilweise erstreckt. Dabei kann die Schale, insbesondere die obere und/oder untere Schale eine Aussparung aufweisen, in der wenigstens eine Strahlungsquelle wenigstens teilweise angeordnet ist.

Die Zentrifuge kann eine Abdeckung aufweisen, die die Strahlungsquelle von dem Rotorraum fluidisch trennt. Dadurch wird verhindert, dass die Strahlungsquelle mit der ausgeschleuderten flüssigen Probe in Kontakt kommt. Die Abdeckung kann derart konfiguriert sein, dass sie Strahlung der Strahlungsquelle transmittiert. So kann die Abdeckung Glas sein, bevorzugt ein Glass mit hoher Transmission im Wellenlängenbereich der Strahlungsquelle, wie z.B. Quarzglas. Die Abdeckung kann bündig zu der Innenfläche des Rotorgehäuses angeordnet sein, sodass sie vom Rotorgehäuse nicht vorsteht.

Die Zentrifuge kann wenigstens eine elektrische Leitung zum elektrischen Verbinden der Strahlungsquelle mit einer Energiequelle aufweisen, wobei die elektrische Leitung wenigstens teilweise durch die vordere Wandung und/oder hintere Wandung verläuft. Dazu weist die vordere und/oder hintere Wandung entsprechende Bohrungen auf. Dabei kann die elektrische Leitung zusätzlich durch die untere und/oder obere Schale verlaufen. Die Energiequelle kann in dem Elektronikraum der Zentrifuge angeordnet sein.

Bei einer besonderen Ausführung kann die Strahlungsquelle derart ausgerichtet sein, dass die Strahlung den Rotor und/oder die Gehäuseinnenfläche des Rotorgehäuses, insbesondere direkt, trifft. Dies bietet den Vorteil, dass Rotorbereiche und/oder Gehäuseinnenflächenbereiche des Rotorgehäuses, bei denen bekannt ist, dass sich die flüssige Probe ablagert, gezielt bestrahlt werden können.

Bei einer Ausführung, bei der die Zentrifuge mehrere Strahlungsquellen aufweist, können wenigstens eine erste Strahlungsquelle oder mehrere erste Strahlungsquellen an oder in der vorderen Wandung und wenigstens eine zweite Strahlungsquelle oder mehrere zweite Strahlungsquellen an oder in der hinteren Wandung angeordnet sein. So können zwei bis vier erste Strahlungsquellen an oder in der vorderen Wandung und zwei bis vier zweite Strahlungsquellen an oder in der hinteren Wanderung vorgesehen werden. Die Anzahl der Strahlungsquellen, die an der vorderen Wandung angebracht sind, kann sich von der Anzahl der Strahlungsquellen unterscheiden, die an der hinteren Wandung angebracht sind.

Die Strahlungsquellen können derart angeordnet sein, dass sie nicht spiegelsymmetrisch zu der Normalebene auf die Rotorwelle zueinander angeordnet sind. Dadurch wird auf einfache Weise erreicht, dass alle Bereiche des Rotors und/oder der Innenfläche des Rotorgehäuses mit Strahlung beaufschlagt werden können.

Die Zentrifuge kann wenigstens zwei Strahlungsquellen aufweisen. Es wurde erkannt, dass wenigstens zwei, insbesondere genau zwei, Strahlungsquellen vorteilhaft sind, weil bei Verwendung einer einzelnen Strahlungsquelle viele Verschattungsbereiche im Rotorgehäuse bestehen. Das Vorsehen wenigstens einer weiteren Strahlungsquelle kann die Verschattungsbereiche reduzieren. Die Strahlungsquellen können bezüglich einer Spiegelebene, die eine Mittelachse der Rotorwelle enthält, zueinander spiegelsymmetrisch angeordnet sein. Die Spiegelebene kann sich entlang einer Gravitationsrichtung erstrecken. Eine derartige Anordnung bietet den Vorteil, dass im Wesentlichen der gesamte oder der gesamte Innenraum des Rotorgehäuses mit Strahlung in kurzer Zeit beaufschlagt werden kann. Dies bietet den Vorteil, dass sichergestellt ist, dass ausgeschleuderte biologische Partikel schnell abgetötet und/oder inaktiviert werden können. Ein kurzer Dekontaminationsbetrieb ist wünschenswert, damit die Zentrifuge zeitnah für einen Waschbetrieb eingesetzt werden kann. Im Dekontaminationsbetrieb werden die biologischen Partikel, wie Mikroorganismen, mit einer Mindeststrahlungsdosis bestrahlt, um einen bestimmten Mindestanteil der Population abzutöten oder zu inaktivieren. Die Dosis kann über die Dauer der Bestrahlung erhöht werden. Um die Mindestbestrahlungszeit zu reduzieren, ist es von Vorteil Verschattungen und Bereiche mit schwacher Intensität zu vermeiden. So kann der Dekontaminationsbetrieb signifikant verkürzt werden.

Die wenigstens zwei Strahlungsquellen können mit der Schale, insbesondere der oberen Schale, verbunden sein. Insbesondere können die wenigstens zwei Strahlungsquellen an oder in der, insbesondere oberen, Schale angeordnet sein. Dabei kann die Schale, insbesondere die obere und/oder die untere Schale, wenigstens zwei Durchbrüche aufweisen. Jeder der Durchbrüche dient zum Aufnehmen wenigstens eines Teils einer Strahlungsquelle und/oder ermöglicht ein Bestrahlen des Rotorraums durch die Strahlungsquelle. Die Strahlungsquellen können derart angeordnet sein, dass die ausgegebene Strahlung in Richtung zur Mittelachse des Rotorgehäuses verläuft. Insbesondere können die Strahlungsquellen derart ausgerichtet sein, dass die ausgestrahlte Strahlung oder ein Großteil der ausgestrahlten Strahlung zuerst auf den Rotor und/oder die Rotorwelle trifft. Die Strahlungsquelle weist einen Öffnungswinkel auf, sodass der Rotor komplett oder im Wesentlichen komplett in Axialrichtung und/oder Radialrichtung bezogen auf die Mittelachse bestrahlt wird. Da der Rotor Durchbrüche aufweist, kann ein Teil der Strahlung durch den Rotor hindurch laufen und die Gehäuseinnenfläche dahinter bestrahlen. Da der Rotor und die Gehäuseinnenfläche aus einem gut reflektierenden Material bestehen und die Oberfläche stark streut, wird die direkte Strahlung von den oben genannten Oberflächen reflektiert und/oder gestreut. Durch eine Rotation des Rotors kann die Strahlung innerhalb des Rotorraums verteilt werden.

Die Strahlungsquellen können in einem Bereich der Schale angeordnet sein, wobei der Bereich der Schale derart angeordnet ist, dass eine Ebene, die senkrecht zur Gravitationsrichtung verläuft und einen Teil des Bereichs der Schale umfasst, die Mittelachse der Rotorwelle enthält oder versetzt zu der Mittelachse der Rotorwelle angeordnet ist. Die Ebene kann dabei in Gravitationsrichtung versetzt zu der Mittelachse angeordnet sein. Dabei kann ein Abstand zwischen der Ebene und der Mittelachse in Gravitationsrichtung in einem Bereich zwischen 0-50%, insbesondere zwischen 1-25%, vorzugsweise 1-15%, des Radialabstandes zwischen der Mittelachse und des Rotorgehäuses betragen. Eine derartige Positionierung der Strahlungsquellen weist den Vorteil auf, dass verhindert wird, dass die Strahlungsquellen in einem Bereich des Rotorgehäuses angeordnet werden, in dem sich die ausgeschleuderte flüssige Probe befindet. Insbesondere wird durch eine derartige Platzierung der Strahlungsquellen verhindert, dass flüssige Probe auf die Abdeckung gelangt und somit Ablagerungen auf der Abdeckung erfolgen können, welche die Effizienz des Dekontaminationsbetriebs beeinträchtigen können. Die oben beschriebene Anordnung der Strahlungsquellen bietet außerdem den Vorteil, dass in dem genannten Bereich die Abdeckung fast senkrecht ist, wodurch sich das Ablagerungsrisiko weiter verringert.

Bei Verwendung von zwei Strahlungsquellen können diese in tangentialer Richtung in einem Bereich zwischen 160° bis 200°, insbesondere 180°, versetzt zueinander angeordnet sein. So wird eine bessere Bestrahlung des Rotorraums erreicht, wenn die Strahlungsquellen möglichst weit voneinander entfernt angeordnet sind. Hingegen können bei Verwendung von drei Strahlungsquellen die Strahlungsquellen in einem Bereich zwischen 100 bis 140°, insbesondere 120°, versetzt zueinander entfernt sein.

Dabei kann eine von wenigstens einer ersten Strahlungsquelle ausgestrahlte Strahlung eine andere Wellenlänge als eine von wenigstens einer zweiten Strahlungsquelle ausgestrahlte Strahlung aufweisen. Dadurch wird erreicht, dass unterschiedliche biologische Partikel besonders effizient abgetötet oder inaktiviert werden. Dies resultiert daraus, dass eine Wellenlänge, bei der ein biologisches Partikel besonders effizient abgetötet oder inaktiviert werden kann, vom Typ der biologischen Partikel abhängen kann.

Wie oben beschrieben ist, weist der Rotor einen Aufnahmeabschnitt auf, in den der nicht zur Zentrifuge gehörende Probenträger eingebracht werden kann. Dabei kann im eingebrachten Zustand des Probenträgers der Rotor mit dem Probenträger drehfest verbunden sein. Dazu kann der Aufnahmeabschnitt eine, insbesondere rechteckige, Basiswand und zwei, insbesondere u-förmige, Schienen aufweisen.

Der Rotor kann außerdem einen weiteren Aufnahmeabschnitt zum Aufnehmen eines weiteren Probenträgers aufweisen. Der weitere Aufnahmeabschnitt kann bezogen auf die Rotorwelle dem Aufnahmeabschnitt diametral gegenüber liegen. Somit kann der Rotor mehrere Probenträger aufnehmen. Zum Aufnehmen des Probenträgers wird der Rotor in eine Aufnahmestellung gedreht, bei der der Probenträger in den jeweiligen Aufnahmeabschnitt des Rotors eingebracht werden kann. Der weitere Aufnahmeabschnitt kann identisch zu dem Aufnahmeabschnitt ausgebildet sein.

Die Zentrifuge kann einen Reflektionskörper aufweisen. Gemäß einer Ausführung kann der Reflektionskörper bei einem Dekontaminationsbetrieb in dem Aufnahmeabschnitt des Rotors eingebracht sein. Dabei kann der Reflektionskörper anstelle des Probenträgers in dem Aufnahmeabschnitt angeordnet sein. Der Reflektionskörper kann wenigstens eine Fläche zum Reflektieren von Strahlung aufweisen. Die Fläche kann eine hohe Reflektanz im Wellenlängenbereich der Strahlungsquelle aufweisen. Insbesondere kann die Fläche eine höhere Reflektanz aufweisen als der Rotor und/oder die Gehäuseinnenfläche. Der Reflektionskörper kann nach dem Dekontaminationsprozess aus dem Rotor ausgebracht werden.

Die Fläche des Reflektionskörpers und/oder der Rotor kann eine Reflektierfläche aufweisen, die derart ausgerichtet ist, dass sie von der Strahlungsquelle ausgegebene Strahlung in einen vorgegebenen Rotorgehäusebereich reflektiert. Gleichermaßen kann zumindest ein Teil der Gehäuseinnenfläche derart ausgerichtet sein, dass sie die von der Strahlungsquelle ausgegebene Strahlung in einen vorgegebenen Rotorgehäusebereich und/oder einen vorgegebenen Rotorbereich reflektiert. Die Gehäuseinnenfläche, insbesondere der zuvor genannte Teil der Gehäuseinnenfläche, kann mit einem Material beschichtet werden, welches eine hohe Reflektanz im Wellenlängenbereich der Strahlungsquelle aufweist. Insbesondere kann das Material eine höhere Reflektanz aufweisen als der nicht beschichtete Rotorgehäusebereich. Darüber hinaus kann durch die Art der Beschichtung auch die Strahlungsrichtung beeinflusst werden, in der reflektierte Strahlung ausgerichtet wird.

Dabei wird als vorgegebener Rotorgehäusebereich und/oder Rotorbereich ein Bereich des Rotorgehäuses bzw. ein Rotorbereich verstanden, der ohne Vorsehen des Reflektionskörpers und/oder der Reflektierfläche des Rotors und/oder des Teils der Gehäuseinnenfläche im Dekontaminationsbetrieb keiner Strahlung ausgesetzt wäre. Somit wird erreicht, dass bislang von der Strahlung nicht erreichbare Bereiche des Rotors und/oder Rotorgehäuses mit Strahlung beaufschlagt werden können.

Bei einer anderen Ausführung kann ein Reflektionskörper drehfest mit dem Rotor verbunden sein. In dieser Ausführung wird der Reflektionskörper nicht in den Aufnahmeabschnitt des Rotors angebracht, sondern ist mit dem Rotor drehfest verbunden, unabhängig davon, ob die Zentrifuge in dem Waschbetrieb oder Dekontaminationsbetrieb betrieben wird. Der Reflektionskörper kann an einer Rotorseite angebracht sein, die in tangentialer Richtung versetzt zu dem Aufnahmeabschnitt, insbesondere zu einer Basiswand, angeordnet ist. Insbesondere kann die Rotorseite zu dem Aufnahmeabschnitt um 90° versetzt angeordnet sein. Dies bedeutet, dass ein Winkel zwischen einer die Rotorseite enthaltenen Ebene und einer den Aufnahmeabschnitt enthaltenen anderen Ebene 90° beträgt.

Der Reflektionskörper kann eine Reflektionsfläche aufweisen. Die Reflektionsfläche kann sich in axialer Richtung der Mittelachse der Rotorwelle erstrecken. Darüber hinaus kann die Reflektionsfläche quer zur Mittelachse der Rotorwelle verlaufen. Dies bedeutet, dass ein radialer Abstand eines Endes der Reflektionsfläche zu der Mittelachse kleiner ist als ein radialer Abstand eines anderen Endes der Reflektionsfläche zu der Mittelachse. Das Vorsehen einer geneigten Reflektionsfläche bietet den Vorteil, dass die Neigung derart gewählt werden kann, dass Bereiche des Rotorgehäuses mit Strahlung versehen werden, die ansonsten nur mit geringer Strahlung beaufschlagt werden. Als "geringe Strahlung" wird verstanden, dass die Strahlungsintensität der dem jeweiligen Bereich zugeführten Strahlung geringer ist als die durchschnittliche Strahlungsintensität und/oder geringer ist als Strahlung, die dem restlichen Bereich des Rotorraums zugeführt wird. Durch Vorsehen des Reflektionskörpers kann erreicht werden, dass die Mindest-Strahlungsintensität erhöht wird, also mehr Strahlung zu den schwer zugänglichen Bereichen gelangt. Dadurch verkürzt sich die Dauer eines Reinigungsschrittes.

Die Zentrifuge kann wenigstens zwei Reflektionskörper aufweisen. Die Reflektionskörper können an sich gegenüberliegenden Rotorseiten angeordnet sein. Die beiden Reflektionskörper können je eine Reflektionsfläche aufweisen. Die Reflektionsflächen können die gleiche Neigung haben. Alternativ können sich die Reflektionsflächen in ihrer Neigung und/oder Neigungsrichtung voneinander unterscheiden. Insbesondere kann ein Reflektionskörper mit einer Rotorseite drehfest verbunden sein und ein anderer Reflektionskörper kann mit einer anderen Rotorseite drehfest verbunden sein. Die beiden Rotorseiten können sich bezüglich der Mittelachse der Rotorwelle gegenüberliegen. Eine derartige Ausführung reflektiert die ausgestrahlte Strahlung besonders gut.

Zusätzlich zu dem Reflektionskörper, der mit dem Rotor drehfest verbunden ist, kann ein Reflektionskörper in den Aufnahmeabschnitt eingebracht werden. Eine derartige Ausführung reflektiert die Strahlung in den Innenraum des Rotorgehäuses besonders gut.

Bei einer besonderen Ausführung kann die wenigstens eine Strahlungsquelle mit dem Rotor drehfest verbunden sein. Somit dreht sich die Strahlungsquelle zusammen mit dem Rotor. Dies bietet den Vorteil, dass ein großer Bereich des Rotorgehäuses bestrahlt werden kann. Dabei kann die am oder im Rotor angeordnete Strahlungsquelle derart ausgerichtet sein, dass die Strahlung vom Rotor radial nach außen ausgegeben wird. Nach außen bedeutet, dass die Strahlung ausgehend vom Rotor zum Rotorgehäuse gerichtet ist.

Der Aufnahmeabschnitt und/oder der weitere Aufnahmeabschnitt können wenigstens eine Strahlungsquelle aufnehmen. Insbesondere kann wenigstens eine Strahlungsquelle am oder im Aufnahmeabschnitt angeordnet sein. Darüber hinaus kann wenigstens eine Strahlungsquelle am oder im weiteren Aufnahmeabschnitt angeordnet sein. Dabei ist eine Ausführung vorteilhaft, bei der die wenigstens eine Strahlungsquelle an oder in der Basiswand und/oder an oder in wenigstens einer der beiden Schienen angeordnet ist. Die Strahlungsquelle kann in einer Aussparung der Basiswand und/oder in einer Aussparung der Schiene angeordnet sein. Dabei kann eine Abdeckung vorhanden sein, die die Strahlungsquelle von dem Rotorraum fluidisch trennt, sodass die Strahlungsquelle nicht in Kontakt mit der ausgeschleuderten flüssigen Probe gelangt.

Die Zentrifuge kann eine elektrische Leitung zum Verbinden der Strahlungsquelle mit einer Energiequelle aufweisen. Die elektrische Leitung kann wenigstens teilweise durch die Rotorwelle verlaufen. Dabei kann ein Schleifenring eingesetzt werden, um die elektrische Energiequelle mit der drehenden Rotorwelle elektrisch zu verbinden.

Bei einer besonderen Ausführung kann die Zentrifuge mehrere Strahlungsquellen aufweisen. Die Strahlungsquellen können in axialer Richtung, insbesondere entlang des Aufnahmeabschnitts und/oder weiteren Aufnahmeabschnitts, beabstandet zueinander angeordnet sind. Darüber hinaus können Strahlungsquellen, insbesondere entlang des Aufnahmeabschnitts und/oder des weiteren Aufnahmeabschnitts, in tangentialer Richtung beabstandet zueinander angeordnet. Die mehreren Strahlungsquellen können sich parallel zu einer Mittelachse der Rotorwelle erstrecken.

Die Strahlungsquelle kann eine punktförmige Strahlungsquelle sein. Alternativ kann die Strahlungsquelle linienförmig ausgeführt sein. Darüber hinaus können mehrere punktförmige Strahlungsquellen entlang einer, insbesondere geraden oder gekrümmten, Linie angeordnet sein. Im Ergebnis kann die Strahlungsquelle oder können die Strahlungsquellen derart angeordnet oder ausgeführt sein, dass ein möglichst großer Bereich, insbesondere der gesamte Bereich, des Rotors und/oder des Rotorgehäuses mit Strahlung beaufschlagt wird.

Die Strahlungsquelle kann eine UV-C Strahlungsquelle sein. Eine UV-C Strahlungsquelle bietet den Vorteil, dass sie biologische Partikel besonders gut abtötet und/oder zersetzt. Dabei kann eine UV-C Strahlungsquelle Strahlung im Wellenlängenbereich zwischen 200nm und 280nm ausgeben.

Die Gehäuseinnenfläche des Rotorgehäuses und/oder eine Rotorfläche und/oder eine Rotorwellenfläche können beschichtet sein. Insbesondere können die Flächen des Rotorgehäuses, des und/oder der Rotorwelle beschichtet werden, die mit flüssiger Probe in Kontakt gelangen können. Vorteilhaft ist, wenn die zuvor genannten Flächen eloxiert sind. Eine eloxierte Fläche weist den Vorteil auf, dass sie die ausgestrahlte Strahlung stärker streut als eine nicht eloxierte Fläche, insbesondere Aluminiumfläche. Ein weiterer Vorteil besteht darin, dass eine eloxierte Fläche chemisch beständiger ist als unbeschichtetes bzw. nicht eloxierte Materialien, wie beispielsweise Aluminium.

Bei einer besonderen Ausführung kann der Strahler zum Ausgeben von Strahlung ein LED-Strahler sein. Der Strahler ist mittels des Wärmeübertragungsabschnitts mit dem Rotorgehäuse wärmeleitend verbunden sein. Die wärmeleitende Verbindung bietet den Vorteil, dass die auftretende Wärme von dem Strahler zu dem Rotorgehäuse abgeführt werden kann. Als Wärmeübertragungsabschnitt wird ein Abschnitt verstanden, der aus festen und/oder flüssigen Komponenten besteht und/oder keine Gasbestandteile aufweist. Dadurch ist eine gute Wärmeabführung von dem Strahler zu dem Rotorgehäuse sichergestellt.

Der Strahler kann an einem metallischen Leitelement des Wärmeübertragungsabschnitts angebracht sein. Der Wärmeübertragungsabschnitt kann eine Leitpaste aufweisen, mittels der das metallische Leitelement mit dem Rotorgehäuse wärmeleitend verbunden ist. Dies bedeutet, dass durch Vorsehen des Wärmeübertragungsabschnitts ermöglicht wird, dass die Wärmeleitung (Konduktion) von dem Strahler zu dem Rotorgehäuse durch nicht gasförmige Komponenten erfolgen kann, wodurch sich die Wärmeabfuhr verbessert.

Die Strahlungsquelle kann außerdem eine Leiterplatte aufweisen. Eine Leiterplatte wird oftmals als PCB (printed circuit board) bezeichnet und dient zum Tragen von elektronischen Bauelementen. Das metallische Leitelement kann mit der Leiterplatte direkt verbunden sein. "Direkt" bedeutet, dass keine weiteren Komponenten zwischen dem metallischen Leitelement und der Leiterplatte angeordnet sind. Dabei kann das metallische Leitelement eine an der Leiterplatte angebrachte Beschichtung, insbesondere Kupferschicht, sein und somit mit der Leiterplatte stoffschlüssig verbunden sein. Alternativ können das metallische Leitelement und die Leiterplatte als separate Komponenten ausgeführt sein. In diesem Fall können die beiden Komponenten mechanisch miteinander verbunden sein. Die Leiterplatte kann mit dem Strahler elektrisch verbunden sein.

Bei einer besonderen Ausführung kann die Steuervorrichtung der Zentrifuge veranlassen, dass während eines Waschbetriebs die Strahlungsquelle keine Strahlung ausgibt. Alternativ oder zusätzlich kann die Steuervorrichtung veranlassen, dass die Strahlungsquelle Strahlung ausgibt, wenn der Rotor keinen Probenträger trägt. In diesem Fall wird somit nur Strahlung ausgegeben, wenn kein Probenträger in dem Aufnahmeabschnitt des Rotors angeordnet ist. Die zuvor beschriebene Steuerung weist den Vorteil auf, dass sichergestellt ist, dass keine in dem Probenträger verbleibenden biologischen Partikel abgetötet werden.

Die Steuervorrichtung kann veranlassen, dass die Strahlung vor oder nach einem Waschbetrieb ausgegeben wird. Dabei kann alternativ oder zusätzlich die Strahlung zu vorgegebenen Zeitpunkten und/oder für eine vorgegebene Zeitdauer ausgegeben werden. So kann der Dekontaminationsbetrieb über Nacht ausgeführt werden, um eine dekontaminierte Zentrifuge zu Beginn jedes Arbeitstages zu erhalten. Darüber hinaus kann die Steuervorrichtung veranlassen, dass die Strahlungsquelle ausgeschaltet wird, sobald die Klappe den Durchgang nicht, insbesondere vollständig, verdeckt.

Darüber hinaus kann die Steuervorrichtung veranlassen, dass der Rotor in dem Dekontaminationsbetrieb in eine vorgegebene Stellung gebracht, insbesondere gedreht, wird, die sich von einer Stellung unterscheidet, bei der der Probenträger in den Rotor einbringbar oder aus dem Rotor ausgebbar ist. Dies bietet den Vorteil, dass der Rotor in eine Stellung gedreht werden kann, bei der der Rotor das Rotorgehäuse wenig abschattet. Bei dieser Ausführung wird der Rotor im Dekontaminationsbetrieb nicht gedreht.

Die Steuervorrichtung kann außerdem veranlassen, dass sich der Rotor während des Dekontaminationsbetriebs dreht. Dabei kann die Steuervorrichtung veranlassen, dass sich der Rotor während der Dauer des gesamten Dekontaminationsbetriebs dreht. Durch die Rotordrehung wird erreicht, dass Strahlung in durch den Rotor ursprünglich verschattete Bereiche des Rotorgehäuses gelangt. Somit wird durch die Rotordrehung erreicht, dass den beispielsweise durch den Rotor verschatteten Bereichen des Rotorgehäuses mehr Strahlung zugeführt wird. Dabei wird der Rotor als Streuer/Reflektor genutzt, der die Strahlung in den Rotationsraum streut/reflektiert. Beim Waschbetrieb kann eine Rotordrehzahl größer sein als in einem Dekontaminationsbetrieb, bei dem Strahlung ausgegeben wird. Eine Drehung des Rotors mit kleiner Drehzahl hat den Vorteil, dass die Strahlung durch den Rotor in einen großen Teil des Rotorgehäuses reflektiert werden kann.

In den Figuren ist der Erfindungsgegenstand schematisch dargestellt, wobei gleiche oder gleichwirkende Elemente zumeist mit denselben Bezugszeichen versehen sind. Dabei zeigt:
- Fig. 1: eine Schnittansicht eines Teils einer erfindungsgemäßen Zentrifuge
- Fig. 2: eine perspektivische Ansicht eines Teils der in Figur 1 gezeigten Zentrifuge ohne hintere Wandung und obere Schale,
- Fig. 3a: eine Draufsicht auf die vordere Wandung,
- Fig. 3b: eine Schnittansicht von oben in einer Ebene, die in der vorderen und hinteren Wand angeordnete Strahlungsquellen aufweist,
- Fig. 4a-f: Schnittansichten eines Rotors der Zentrifuge in unterschiedlichen Ausführungsformen,
- Fig. 5: eine perspektivische Darstellung eines Teils einer erfindungsgemäßen Zentrifuge mit anderer Anordnung der Strahlungsquellen,
- Fig. 6: eine Schnittansicht auf einen Teil der in Fig. 5 dargestellten Zentrifuge von vorne,
- Fig. 7: eine perspektivische Darstellung einer oberen Schale der in fig. 5 dargestellten Zentrifuge
- Fig. 8: eine vergrößerte Darstellung einer Strahlungsquelle;
- Fig. 9: eine perspektivische Darstellung auf einen Rotor;
- Fig. 10: eine perspektivische Ansicht eines Teils der Zentrifuge mit Elektronikraum,
- Fig. 11: eine perspektivische Ansicht der Zentrifuge.

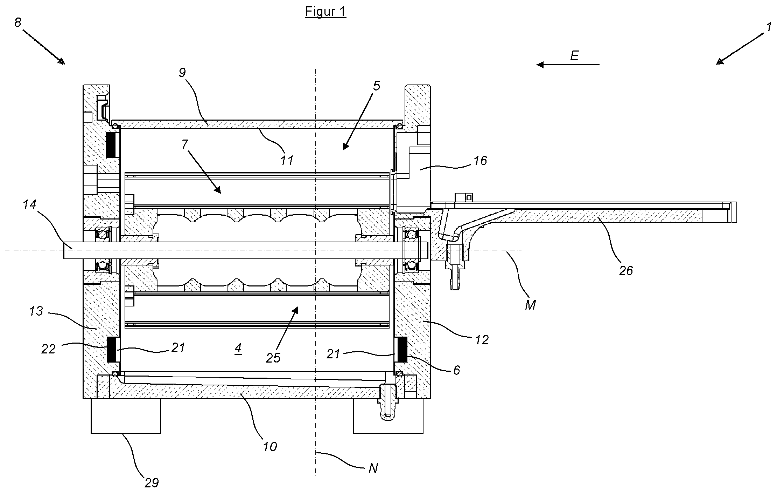

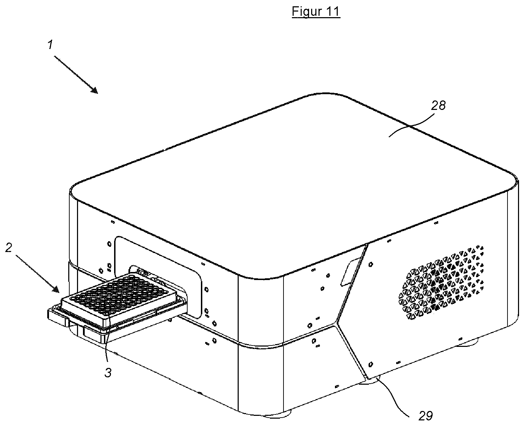

Eine in Figur 1 gezeigte Zentrifuge 1 dient zum Rotieren eines in Figur 11 gezeigten Probenträgers 2. Die Zentrifuge 1 weist einen Rotor 5 auf, der einen Aufnahmeabschnitt 7 zum Aufnehmen des Probenträgers 2 aufweist. Darüber hinaus weist die Zentrifuge 1 einen Rotorraum 4, in welchem der Rotor 5 angeordnet ist, und mehrere Strahlungsquellen auf, nämlich eine erste Strahlungsquelle 6 und eine zweite Strahlungsquelle 22, zum Ausgeben einer Strahlung in den Rotorraum 4.

Die Strahlungsquellen 6, 22 sind derart ausgebildet, dass sie eine Strahlung mit einer Wellenlänge von höchstens 350nm (Nanometer) ausgeben. Insbesondere können die Strahlungsquellen 6, 22 derart ausgebildet sein, dass sie Strahlung mit einer Wellenlänge im Bereich zwischen 100nm, insbesondere 200nm, bis 350nm, insbesondere 315, vorzugsweise 280, ausgeben. Ganz besonders vorteilhaft sind Strahlungsquellen 6, 22, die eine Strahlung mit einer Wellenlänge von 254nm oder in einem Bereich zwischen 260nm bis 265nm, ausgibt. Die ersten Strahlungsquellen 6 und die zweiten Strahlungsquellen 22 können Strahlungen mit unterschiedlicher Wellenlänge ausgeben. Dabei können die Strahlungsquellen 6, 22 jeweils eine UV-C Strahlungsquelle sein.

Die ersten Strahlungsquellen 6 können in der vorderen Wandung 12 angeordnet sein. Insbesondere kann die vordere Wandung 12 Aussparungen aufweisen, in denen jeweils eine erste Strahlungsquellen 6 angeordnet ist. Die Zentrifuge 1 kann eine Abdeckung 21 aufweisen, dass die erste Strahlungsquelle 6 von dem Rotorraum 4, insbesondere fluidisch trennt. Die Abdeckung 21 ist ebenfalls in der Aussparung angeordnet. Die zweiten Strahlungsquellen 22 können in der hinteren Wandung 13 angeordnet sein. Insbesondere kann die hintere Wandung 13 Aussparungen aufweisen, in denen jeweils eine zweite Strahlungsquelle 22 angeordnet ist. Die Zentrifuge 1 kann eine Abdeckung 21 aufweisen, die die zweite Strahlungsquelle 22 von dem Rotorraum 4, insbesondere fluidisch trennt.

Der Rotorraum 4 wird durch ein Rotorgehäuse 8 der Zentrifuge 1 begrenzt. Das Rotorgehäuse 8 weist eine obere Schale 9 und eine untere Schale 10 auf. Beide Schalen 9, 10 sind lösbar miteinander verbunden. Darüber hinaus weist das Rotorgehäuse 8 die vordere Wandung 12 und die hintere Wandung 13 auf. Dabei begrenzt nur ein Teil der vorderen Wandung 12 und ein Teil der hinteren Wandung 13 den Rotorraum 4.

Die Strahlungsquellen 6, 22 sind derart ausgerichtet, dass sie Strahlung in den Rotorraum 4 ausgeben. Dabei sind die Strahlungsquellen 6, 22 derart ausgerichtet, dass die ausgegebene Strahlung an dem Rotor 5 und/oder einer Gehäuseinnenfläche 11 reflektiert. Die Gehäuseinnenfläche 11 wird durch die zu dem Rotorraum 4 weisenden Flächen der oberen Schale 9, der unteren Schale 10, der vorderen Wandung 12 und der hinteren Wandung 13 gebildet. Dabei ist die Gehäuseinnenfläche 11 in einer Normalebene N, die senkrecht zu einer Mittelachse M steht und einen Teil der oberen Schale 9 und der unteren Schale 10 umfasst, zylinderförmig ausgebildet.

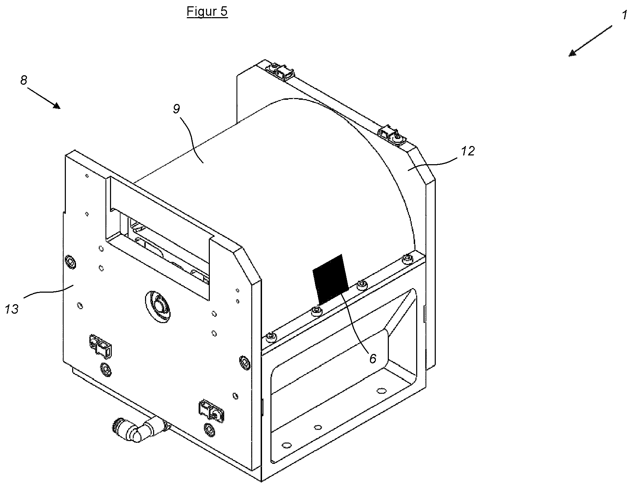

Die Zentrifuge 1 weist eine Zentrifugenaufnahme 26 auf, auf die ein Nutzer der Zentrifuge 1 den Probenträger 2 setzt. Eine in den Figuren nicht dargestellte Verschiebeeinrichtung bewegt dann den Probenträger 2 in den Aufnahmeabschnitt 7 des Rotors 5 entlang einer Einbringrichtung E oder bewegt den Probenträger 2 aus dem Aufnahmeabschnitt 7 in die Zentrifugenaufnahme 26 entlang einer Ausbringrichtung, die entgegengesetzt zu der Einbringrichtung E ist. Die Zentrifugenaufnahme 26 steht von der vorderen Wandung 12, insbesondere in axialer Richtung, vor. Die Verschiebeeinrichtung wird durch einen nicht dargestellten Verschiebemotor angetrieben, der in dem in Figur 5 gezeigten Elektronikraum angeordnet ist. Die Verschiebeeinrichtung kann ein gewickeltes Band aufweisen, das durch den Verschiebemotor angetrieben wird. Zum Koppeln mit dem in der Zentrifugenaufnahme 26 angeordneten Probenträger 2 bewegt sich die Verschiebeeinrichtung durch den Rotorraum 4 hindurch und wird mit dem Probenträger 2, insbesondere magnetisch, verbunden.

Die vordere Wandung 12 weist einen Durchgang 16 auf, durch den der Probenträger 2 in den Aufnahmeabschnitt 7 des Rotor 5 eingebracht werden kann. Der Probenträger 2 wird durch den Durchgang 16 aus dem Aufnahmeabschnitt 7 ausgebracht. Der Durchgang 16 ist bezüglich einer Mittelachse M des Rotorgehäuses 8 radial versetzt angeordnet. Insbesondere ist der Durchgang 16 derart ausgebildet, dass er vollständig versetzt zu der Mittelachse M angeordnet ist, also die Mittelachse M durch den Durchgang 16 nicht verläuft.

Der Rotor 5 ist mit einer Rotorwelle 14 drehfest verbunden. Die Rotorwelle 14 ist in der vorderen Wandung 12 und der hinteren Wanderung 13 jeweils drehbar gelagert und erstreckt sich durch die hintere Wandung 13 hindurch. Dabei ist die Rotorwelle 14 mit einem in den Figuren nicht dargestellten Motor triebtechnisch verbunden. Der Motor ist in einem in Figur 5 dargestellten Elektronikraum 17 angeordnet.

Bei der in Figur 1 dargestellten Stellung ist der Rotor 5 derart gedreht, dass ein Probenträger 2 in den Aufnahmeabschnitt 7 eingebracht werden kann. Der Rotor 5 weist einen weiteren Aufnahmeabschnitt 25 auf, der bezogen auf die Mittelachse M dem Aufnahmeabschnitt diametral gegenüberliegt. Der weitere Aufnahmeabschnitt 25 ist identisch ausgebildet wie der Aufnahmeabschnitt 7. Insofern kann der Rotor 5 zwei Probenträger 2 aufnehmen.

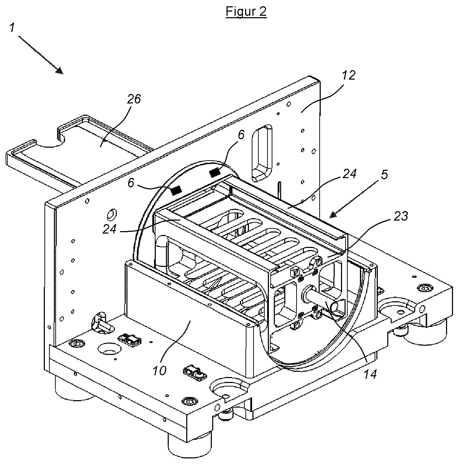

Fig. 2 zeigt eine perspektivische Ansicht eines Teils der in Figur 1 gezeigten Zentrifuge 1 ohne hintere Wandung und obere Schale. Die untere Schale 10 weist in der Normalebene N eine halbzylinderförmige Gehäuseinnenfläche 11 auf. Nach Aufsetzen der oberen Schale 9 ist die Gehäuseinnenfläche 11 in der Normalebene N zylinderförmig ausgebildet.

Der Aufnahmeabschnitt 7 weist eine Basiswand 23 und zwei u-förmige Schienen 24 auf. Nach Einbringen des Probenträgers 2 in den Aufnahmeabschnitt 7 liegt der Probenträger 2 auf der Basiswand 23 auf. Die u-förmigen Schienen 24 verhindern ein Bewegen des Probenträgers 2 in radialer Richtung.

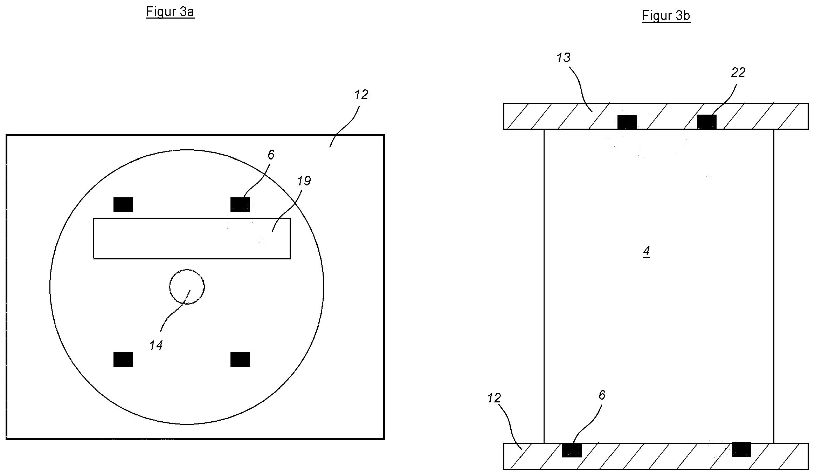

Fig. 3a zeigt eine Draufsicht auf die vordere Wandung 12. Die vordere Wandung 12 weist vier erste Strahlungsquellen 6 auf. Die ersten Strahlungsquellen 6 sind in tangentialer Richtung bezogen auf die Mittelachse M zueinander beabstandet angeordnet. Darüber hinaus weist die vordere Wandung 12 den Durchgang 16 auf, die mittels einer Klappe 19 verschlossen werden kann.

Figur 3b zeigt eine Schnittansicht von oben in einer Ebene, insbesondere einer Horizontalebene, die in der vorderen Wandung 12 und hinteren Wandung 13 angeordnete Strahlungsquellen 6, 22 aufweist. Dabei ist in der Figur 3b der Rotor 5 nicht dargestellt, sondern nur der Rotorraum 4 ersichtlich. Auch wenn dies aus Figur 3b nicht ersichtlich ist, weist die hintere Wandung 13 vier zweite Strahlungsquellen 22 auf. Die zweiten Strahlungsquellen 22 sind in tangentialer Richtung bezogen auf die Mittelachse M zueinander beanstandet angeordnet. Die ersten und zweiten Strahlungsquellen 6, 22 sind derart angeordnet, dass sie bezogen auf eine in Figur 1 dargestellten Normalebene N nicht spiegelsymmetrisch zueinander angeordnet sind. Dies bedeutet, dass die Strahlungsquellen versetzt zueinander angeordnet sind, sodass ein großer Teil der Innenfläche 11 des Rotorgehäuses 8 bestrahlt werden kann. Bei alternativen, nicht dargestellten Ausführungen können die Strahlungsquellen bezogen auf die Normalebene N spiegelsymmetrisch zueinander angeordnet sein.

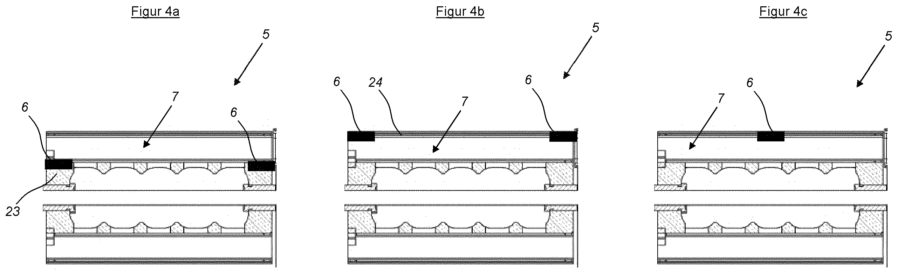

Die Fig. 4a-f zeigen Schnittansichten eines Rotors 5 der Zentrifuge 1 in unterschiedlichen Ausführungsformen. Bei der in Figur 4a dargestellten Ausführung des Rotors 5 sind zwei erste Strahlungsquellen 6 in der Basiswand 23 des Rotor 5 angeordnet. Die Strahlungsquellen 6 sind jeweils an einem Ende des Rotors 5 angeordnet. Darüber hinaus sind die Strahlungsquellen 6 in axialer Richtung beabstandet zueinander angeordnet.

Bei der in Fig. 4b dargestellten Ausführung des Rotors 5 sind zwei erste Strahlungsquellen 6 in wenigstens einer der beiden Schienen 24 angeordnet. Die Strahlungsquellen 6 sind jeweils an einem Ende des Rotors 5 angeordnet. Darüber hinaus sind die Strahlungsquellen 6 in axialer Richtung beabstandet zueinander angeordnet.

Bei der in Fig. 4c dargestellten Ausführung des Rotors 5 ist eine erste Strahlungsquelle 6 in wenigstens einer der beiden Schienen 24 angeordnet. Die erste Strahlungsquelle 6 weist einen größeren Ausstrahlungswinkel auf als die Strahlungsquellen bei den in den Fig. 4a und 4b dargestellten Ausführungen.

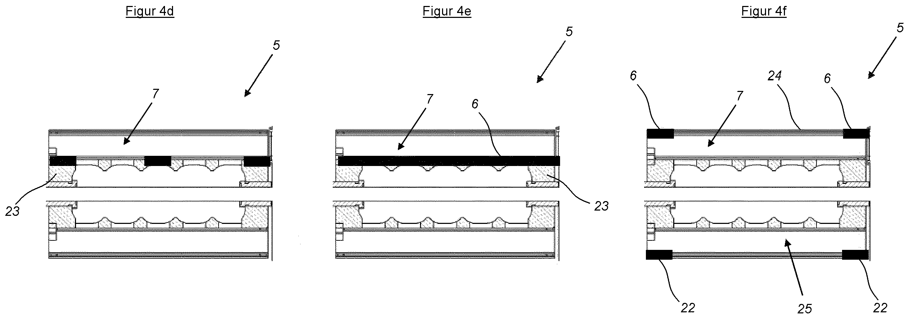

Die in Fig. 4d dargestellte Ausführung des Rotors 5 unterscheidet sich von der in Fig. 4a dargestellten Ausführung darin, dass drei erste Strahlungsquellen 6 vorhanden ist. Die dritte Strahlungsquelle 6 ist ebenfalls in der Basiswand 23 angeordnet.

Die in der Fig. 4e dargestellte Ausführung des Rotor 5 weist eine linienförmige Strahlungsquelle 6 auf, die sich in axialer Richtung entlang der gesamten Länge der Basiswand 23 erstreckt.

Die in Fig. 4f dargestellte Ausführung des Rotors 5 unterscheidet sich von der in Figur 4b dargestellten Ausführung darin, dass weitere Strahlungsquellen 22 vorhanden sind. Die zweiten Strahlungsquellen 22 sind in dem weiteren Aufnahmeabschnitt 25 angeordnet. Insbesondere sind die zweiten Strahlungsquellen 22 in wenigstens einer der Schienen 24 des weiteren Aufnahmeabschnitts 25 angeordnet. Die zweiten Strahlungsquellen 22 sind jeweils an einem Ende des Rotors 5 angeordnet. Darüber hinaus sind die zweiten Strahlungsquellen 22 in axialer Richtung beabstandet zueinander angeordnet.

Die in den Fig. 4a bis 4f dargestellten Rotoren 5 können in der in den Figuren 1, 2, 5, 11 und 12 dargestellten Zentrifuge 1 eingesetzt werden.

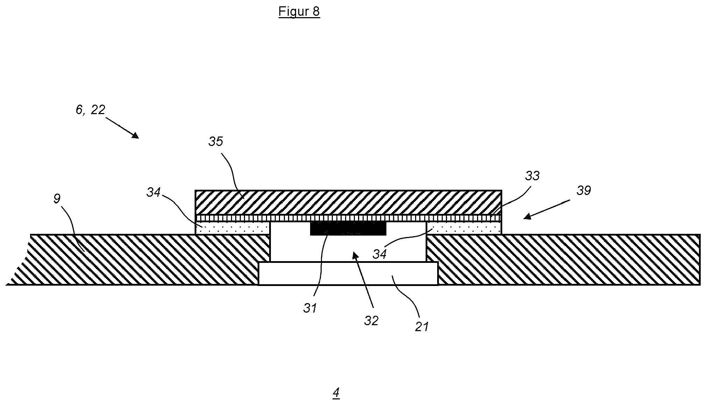

Fig. 5 zeigt eine perspektivische Darstellung eines Teils einer erfindungsgemäßen weiteren Zentrifuge 1. Die Zentrifuge 1 unterscheidet sich von den oben beschriebenen Zentrifugen 1 in der Anordnung der Strahlungsquellen 6, 22. So ist bei der in Figur 5 dargestellten Ausführung eine erste Strahlungsquelle 6 mit der oberen Schale 9 befestigt. Eine nicht dargestellte zweite Strahlungsquelle 22 ist ebenfalls mit der oberen Schale 9 befestigt. Der Aufbau der ersten und zweiten Strahlungsquelle 6, 22 ist in Figur 8 gezeigt. Die erste und zweite Strahlungsquelle 6, 22 sind identisch ausgebildet.

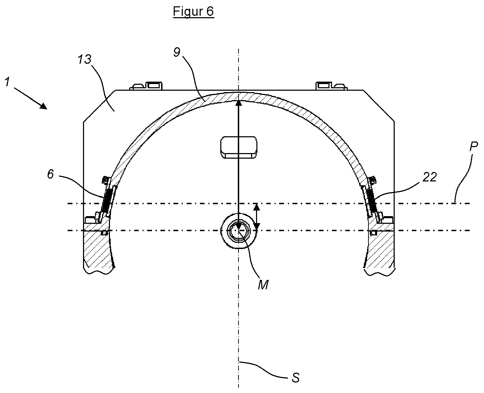

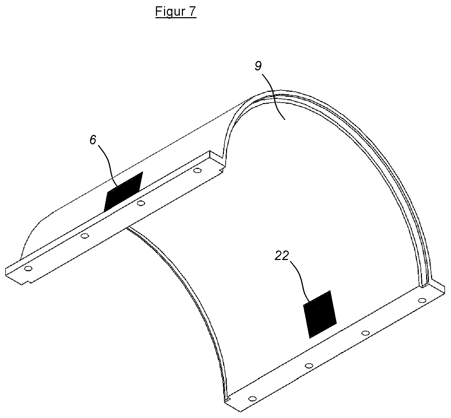

Fig. 6 zeigt eine Schnittansicht auf den in Fig. 5 dargestellten Teil der Zentrifuge 1 von vorne und Fig. 7 zeigt eine perspektivische Darstellung der oberen Schale 9 der in Fig. 5 dargestellten weiteren Zentrifuge 1 Die Zentrifuge 1 weist zwei Strahlungsquellen 6, 22 auf. Beide Strahlungsquellen 6, 22 sind mit der oberen Schale 9 befestigt. Wie aus Figur 8 besser ersichtlich ist, sind die beiden Strahlungsquellen 6, 22 an der oberen Schale 9 angeordnet und/oder sind teilweise in einem Durchbruch 32 der oberen Schale 9 angeordnet. Dabei sind die beiden Strahlungsquellen bezüglich einer Spiegelebene S spiegelsymmetrisch zueinander angeordnet. Die Spiegelebene S weist die Mittelachse M des Rotorgehäuses 8 auf und erstreckt sich außerdem in Gravitationsrichtung. Die beiden Strahlungsquellen 6, 22 sind derart ausgerichtet, dass die ausgestrahlte Strahlung in Richtung zur Mittelachse M des Rotorgehäuses 8 verläuft. Sprich die ausgestrahlte Strahlung trifft den nicht dargestellten Rotor 5 bevor sie durch eine Gehäuseinnenfläche 11 reflektiert wird.

Darüber hinaus ist aus Figur 6 ersichtlich, dass die beiden Strahlungsquellen 6, 22 in einem Bereich der oberen Schale 6 derart angeordnet ist, dass eine Ebene P existiert, deren radialer Abstand zur Mittelachse M, also in Gravitationsrichtung, kleiner ist als 50% des Radialabstands zwischen der Mittelachse M und dem Rotorgehäuse 8. Als Radialabstand zwischen der Mittelachse M und dem Rotorgehäuse 8 wird der Abstand in Gravitationsrichtung verstanden. Die Ebene P verläuft senkrecht zur Gravitationsrichtung und enthält einen Teil der beiden Strahlungsquellen 6, 22. In Figur 6 ist sowohl der radiale Abstand zwischen der Mittelachse M und der Ebene P als auch der radiale Abstand zwischen der Mittelachse M und dem Rotorgehäuse 8 als Doppelpfeil symbolisiert. Die beiden Strahlungsquellen 6, 22 sind in tangentialer Richtung in einem Bereich zwischen 160° bis 200°, vorzugsweise 180° versetzt zueinander angeordnet.

Fig. 8 zeigt eine vergrößerte Darstellung einer Strahlungsquelle 6, 22. Die Strahlungsquelle 6, 22 weist einen Strahler 31, wie eine LED, auf, mittels der Strahlung in den Rotorraum 4 ausgestrahlt wird. Die Strahlungsquelle 6, 22 ist an der oberen Schale 9 angeordnet, wobei sich der Strahler 31 teilweise in einen Durchbruch 32 der oberen Schale 9 erstreckt.

Die Strahlungsquelle 6, 22 weist eine Wärmeübertragungsabschnitt 39 auf, mittels dem der Strahler 31 mit der oberen Schale 9 wärmeleitend verbunden ist. Der Wärmeübertragungsabschnitt 39 weist ein metallisches Leitelement 33 auf, wobei der Strahler 31 an dem metallischen Leitelement 33 angeordnet ist. Darüber hinaus weist die Strahlungsquelle 6, 22 eine Leiterplatte 35 auf. Das metallische Leitelement 33 ist mit der Leiterplatte 35 stoffschlüssig verbunden. Darüber hinaus ist das metallische Leitelement 33 mittels Leitpasten 34 direkt mit der oberen Schale 9 verbunden. Im Ergebnis erfolgt eine Wärmekonduktion von dem Strahler 31 zu der oberen Schale 9 nur über nicht gasförmige Abschnitte des Wärmeübertragungsabschnitts 39.

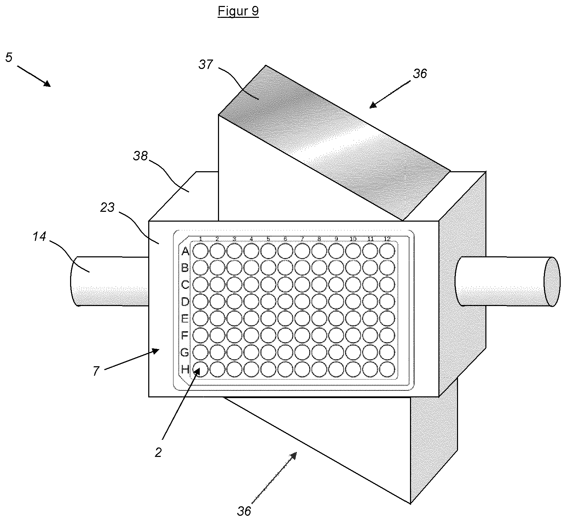

Fig. 9 zeigt eine perspektivische Darstellung auf einen Rotor 5. Der Rotor 5 unterscheidet sich von dem Rotor, der in den in Figuren 1 bis 8 dargestellten Ausführungen eingesetzt wird, dadurch, dass der Rotor 5 zwei Reflektionskörper 36 aufweist. Beide Reflektionskörper 36 sind jeweils an einer Rotorseite 38 angeordnet. Die Rotorseite 38 ist in tangentialer Richtung versetzt zu dem Aufnahmeabschnitt 7 zum Aufnehmen des Probenträgers 2 vorgesehen. Insbesondere verläuft die Rotorseite 38 senkrecht oder im Wesentlichen senkrecht zu der Basiswand 23 des Rotors 5.

Die beiden Reflektionskörper 36 sind an sich gegenüberliegenden Rotorseiten 38 angeordnet. Die beiden Rotorseiten 38 liegen sich bezüglich der Mittelachse der Rotorwelle 14, insbesondere radial, gegenüber. Beide Reflektionskörper 36 weisen jeweils eine Reflektionsfläche 37, wie beispielsweise einen Spiegel, auf, mittels der die Strahlung reflektiert wird. Die Reflektionsfläche 37 weist eine höhere Reflektanz auf als die weiteren Bauteile des Rotors 5 und/oder Rotorwelle 14. Die Reflektionsfläche 37 verläuft quer zu einer der Mittelachse der Rotorwelle 14.

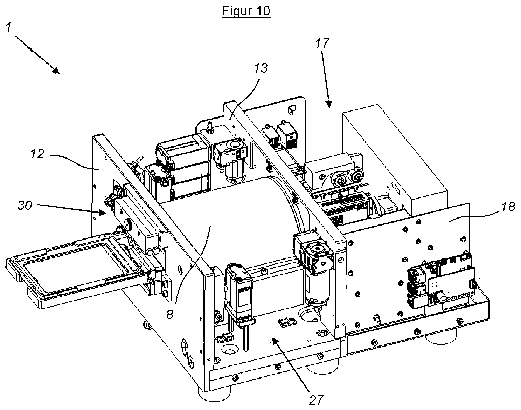

Fig. 10 zeigt eine perspektivische Ansicht eines Teils der Zentrifuge 1 mit Elektronikraum 17. Der Elektronikraum 17 ist durch die hintere Wandung 13 von dem Rotorraum 4 und einem Vorderraum 27 der Zentrifuge 1 getrennt. In dem Elektronikraum 17 ist eine Steuervorrichtung 18 zum Steuern eines Waschbetriebs und/oder eines Dekontaminationsbetriebs angeordnet. Darüber hinaus ist in dem Elektronikraum 17 die Verschiebeeinrichtung zum Einbringen oder Ausbringen des Probenträgers 2 in den Rotor 5 oder aus dem Rotor 5 und der Verschiebemotor angeordnet. Das Rotorgehäuse 8 ist in dem Vorderraum 27 angeordnet. Darüber hinaus kann die Zentrifuge 1 eine Dispensiervorrichtung 30 aufweisen, die an der vorderen Wand 12 angeordnet ist.

Das Rotorgehäuse 8, der Rotor 5 und die Strahlungsquellen 6, 22 können analog zu den in den Figuren 1- 9 beschriebenen Ausführungen ausgebildet bzw. angeordnet sein.

Fig. 11 zeigt eine perspektivische Ansicht der Zentrifuge 1. Die Zentrifuge 1 weist ein Zentrifugengehäuse 28 auf, das den Vorderraum 27 und den Elektronikraum 17 umschließt. Das Rotorgehäuse 8 ist ebenfalls in einem durch das Zentrifugengehäuse 28 umschlossenen Raum angeordnet. Darüber hinaus weist die Zentrifuge 1 Standfüße 29 zum Aufstellen der Zentrifuge 1 auf einen Boden oder Arbeitstisch oder dergleichen auf. Der Probenträger 2 ist als Mikrotiterplatte ausgeführt, die in der Zentrifugenaufnahme 26 angeordnet ist. Die Mikrotiterplatte weist eine Vielzahl von Behältnissen 3 auf.

Die Zentrifuge 1 kann analog zu einer in den Figuren 1 bis 10 beschriebenen Ausführungen ausgeführt sein.

## Patentansprüche

1. Zentrifuge (1) zum Rotieren eines Probenträgers (2), der wenigstens ein Behältnis (3) zum Aufnehmen einer flüssigen Probe aufweist, mit einem drehbaren Rotor (5), der wenigstens einen Aufnahmeabschnitt (7) zum Aufnehmen des Probenträgers (2) aufweist, und einem Rotorraum (4), in welchem der Rotor (5) angeordnet ist, wobei die Zentrifuge (1) wenigstens eine Strahlungsquelle (6, 22) zum Ausgeben einer Strahlung in den Rotorraum (4) mit einer Wellenlänge von höchstens 350nm aufweist, wobei der Rotorraum (4) durch ein Rotorgehäuse (8) begrenzt ist, **dadurch gekennzeichnet, dass** die Strahlungsquelle (6, 22) einen Strahler (31) aufweist, der mittels eines Wärmeübertragungsabschnitts (39) mit dem Rotorgehäuse (8) wärmeleitend verbunden ist.

2. Zentrifuge (1) nach Anspruch 1, **dadurch gekennzeichnet, dass**
a. die wenigstens eine Strahlungsquelle (6, 22) am oder im Rotorgehäuse (8) angeordnet ist und/oder dass
b. eine vordere Wandung (12) und/oder eine hintere Wandung (13) des Rotorgehäuses (8) eine Aussparung aufweist, in der wenigstens eine Strahlungsquelle (6, 22) angeordnet ist und/oder dass
c. eine obere und/oder eine untere Schale (9, 10) des Rotorgehäuses (8) eine Aussparung aufweist, in der wenigstens eine Strahlungsquelle (6, 22) angeordnet ist.

3. Zentrifuge (1) nach Anspruch 1 oder 2, **dadurch gekennzeichnet, dass**
a. die Zentrifuge (1) eine Abdeckung (21) aufweist, die die Strahlungsquelle (6, 22) von dem Rotorraum (4) fluidisch trennt und/oder derart ausgebildet ist, dass sie Strahlung der Strahlungsquelle (6, 22) transmittiert und/oder dass
b. die Zentrifuge (1) wenigstens eine elektrische Leitung zum elektrischen Verbinden der Strahlungsquelle (6, 22) mit einer Energiequelle aufweist, wobei die elektrische Leitung wenigstens teilweise durch die vordere Wandung (12) und/oder hintere Wandung (13) verläuft und/oder dass
c. die Strahlungsquelle (6, 22) derart ausgerichtet ist, dass die Strahlung den Rotor (5) und/oder eine Gehäuseinnenfläche (11) trifft.

4. Zentrifuge (1) nach Anspruch 2 oder 3, **dadurch gekennzeichnet, dass** mehrere Strahlungsquellen (6, 22) vorhanden sind, wobei eine von wenigstens einer ersten Strahlungsquelle (6) ausgestrahlte Strahlung eine andere Wellenlänge als eine von wenigstens einer zweiten Strahlungsquelle (22) ausgestrahlte Strahlung aufweist.

5. Zentrifuge (1) nach einem der Ansprüche 1 bis 4, **dadurch gekennzeichnet, dass** wenigstens zwei Strahlungsquellen bezüglich einer Spiegelebene (S), die eine Mittelachse der Rotorwelle (14) enthält, zueinander spiegelsymmetrisch angeordnet sind.

6. Zentrifuge (1) nach einem der Ansprüche 1 bis 5 **dadurch gekennzeichnet, dass** die Zentrifuge (1) wenigstens einen Reflektionskörper aufweist, der in dem Aufnahmeabschnitt (7) des Rotors (4) eingebracht ist, wobei der Reflektionskörper wenigstens eine Fläche zum Reflektieren von Strahlung aufweist.

7. Zentrifuge (1) nach einem der Ansprüche 1 bis 6, **dadurch gekennzeichnet, dass**
a. der Rotor (4) eine Reflektierfläche aufweist, die derart ausgerichtet ist, dass sie die von der Strahlungsquelle (6, 22) ausgegebene Strahlung in einen vorgegebenen Gehäusebereich reflektiert und/oder dass
b. ein Teil der Gehäuseinnenfläche (11) derart ausgerichtet ist, dass sie die von der Strahlungsquelle (6, 22) ausgegebene Strahlung in einen vorgegebenen Rotorgehäusebereich und/oder einen vorgegebenen Rotorbereich reflektiert.

8. Zentrifuge (1) nach einem der Ansprüche 1 bis 7, **dadurch gekennzeichnet, dass** die Zentrifuge (1) wenigstens einen Reflektionskörper (36) aufweist, wobei
a. der Reflektionskörper (36) drehfest mit dem Rotor (5) verbunden ist und/oder dass
b. der Reflektionskörper (36) an einer Rotorseite (38) angebracht ist, die in tangentialer Richtung versetzt zu dem Aufnahmeabschnitt (7) angeordnet ist und/oder dass
c. der Reflektionskörper (36) eine Reflektionsfläche (37) aufweist, die sich axial erstreckt und/oder quer zu der Mittelachse der Rotorwelle (14) verläuft und/oder dass
d. die Zentrifuge (1) wenigstens zwei Reflektionskörper (36) aufweist, die an sich gegenüberliegenden Rotorseiten (38) angeordnet sind.

9. Zentrifuge (1) nach einem der Ansprüche 1 bis 8, **dadurch gekennzeichnet, dass** die wenigstens eine Strahlungsquelle (6, 22) mit dem Rotor (5) drehfest verbunden ist.

10. Zentrifuge (1) nach Anspruch 9, **dadurch gekennzeichnet, dass**
a. die Strahlungsquelle (6. 22) derart ausgerichtet ist, dass die Strahlung vom Rotor (5) radial nach außen ausgegeben wird und/oder dass
b. die Zentrifuge (1) eine elektrische Leitung zum Verbinden der Strahlungsquelle (6, 22) mit einer Energiequelle aufweist, wobei die elektrische Leitung wenigstens teilweise durch die Rotorwelle (14) verläuft.

11. Zentrifuge (1) nach einem der Ansprüche 1 bis 10, **dadurch gekennzeichnet, dass**
a. der Strahler (31) an einem metallischen Leitelement (33) des Wärmeübertragungsabschnitts (39) angebracht ist und/oder dass
b. der Wärmeübertragungsabschnitt (39) eine Leitpaste (34) aufweist, mittels der ein metallisches Leitelement (33) der Wärmeübertragungsabschnitt (39) mit dem Rotorgehäuse (8) wärmeleitend verbunden ist und/oder dass
c. die Strahlungsquelle (6, 22) eine Leiterplatte (35) aufweist, die direkt mit dem metallischen Leitelement (33) verbunden ist.

12. Zentrifuge (1) nach einem der Ansprüche 1 bis 11, **dadurch gekennzeichnet, dass** die Zentrifuge (1) eine Steuervorrichtung (18) aufweist, die veranlasst, dass
a. während eines Waschbetriebs die Strahlungsquelle (6, 22) keine Strahlung ausgibt und/oder dass
b. die Strahlungsquelle (6, 22) Strahlung ausgibt, wenn kein Probenträger (2) im Rotor (5) angeordnet ist und/oder dass
c. der Rotor (5) in einem Dekontaminationsbetrieb in eine vorgegebene Stellung gebracht wird, die sich von einer Stellung unterscheidet, bei der der Probenträger (2) in den Rotor (5) einbringbar oder aus dem Rotor (5) ausgebbar ist und/oder dass
d. der Rotor (5) während des, Dekontaminationsbetrieb gedreht wird.

13. Verfahren beim Betreiben einer Zentrifuge (1) nach einem der vorhergehenden Ansprüche 1 bis 12, wobei ein Probenträger (2), der wenigstens ein Behältnis (3) zum Aufnehmen einer flüssigen Probe (5) aufweist, mittels eines Rotors (5) in einem Rotorraum (4) gedreht wird, **dadurch gekennzeichnet, dass** eine Strahlung mit einer Wellenlänge von höchstens 350nm in den Rotorraum (4) ausgegeben wird.

14. Verfahren nach Anspruch 13, **dadurch gekennzeichnet, dass**
a. die Strahlung zeitlich vor oder nach einem Waschbetrieb ausgegeben wird und/oder dass
b. die Strahlung ausgegeben wird, wenn der Rotor (5) keinen Probenträger (2) aufnimmt und/oder dass
c. die Strahlung zu vorgegebenen Zeitpunkten und/oder für eine vorgegebene Zeitdauer ausgegeben wird.

15. Verfahren nach Anspruch 13 oder 14, **dadurch gekennzeichnet, dass**
a. der Rotor (5) während des Dekontaminationsbetriebs gedreht wird und/oder dass
b. eine Rotordrehzahl beim Waschbetrieb größer ist als bei einem Dekontaminationsbetrieb, bei dem Strahlung ausgegeben wird und/oder dass
c. der Rotor (5) in einem Dekontaminationsbetrieb in eine Stellung gedreht wird, die sich von einer Stellung unterscheidet, bei der der Probenträger (2) in den Rotor (5) eingebracht oder aus dem Rotor (5) ausgebracht wird und/oder dass
d. die Strahlungsquelle (6, 22) ausgeschaltet wird, sobald die Klappe (19) den Durchgang (16) nicht verdeckt.

## Claims

1. A centrifuge (1) for rotating a sample carrier (2) having at least one container (3) for receiving a liquid sample, having a rotatable rotor (5) having at least one receiving section (7) for receiving the sample carrier (2), and a rotor chamber (4), in which the rotor (5) is arranged, wherein the centrifuge (1) has at least one radiation source (6, 22) for emitting radiation into the rotor chamber (4) with a wavelength of at most 350 nm, wherein the rotor chamber (4) is delimited by a rotor housing (8), **characterized in that** the radiation source (6, 22) has an emitter (31) which is connected to the rotor housing (8) in a thermally conductive manner by means of a heat transfer section (39).

2. The centrifuge (1) according to claim 1, **characterized in that**
a. the at least one radiation source (6, 22) is arranged on or in the rotor housing (8) and/or **in that**
b. a front wall (12) and/or a rear wall (13) of the rotor housing (8) has a recess in which at least one radiation source (6, 22) is arranged and/or **in that**
c. an upper and/or a lower shell (9, 10) of the rotor housing (8) has a recess in which at least one radiation source (6, 22) is arranged.

3. The centrifuge (1) according to claim 1 or 2, **characterized in that**
a. the centrifuge (1) has a cover (21) which fluidically separates the radiation source (6, 22) from the rotor chamber (4) and/or is designed in such a way that it transmits radiation from the radiation source (6, 22) and/or **in that**
b. the centrifuge (1) has at least one electrical line for the electrical connection of the radiation source (6, 22) to an energy source, wherein the electrical line runs at least partially through the front wall (12) and/or rear wall (13) and/or **in that**
c. the radiation source (6, 22) is oriented in such a way that the radiation is incident on the rotor (5) and/or an inner housing surface (11).

4. The centrifuge (1) according to claim 2 or 3, **characterized in that** a plurality of radiation sources (6, 22) are present, wherein radiation emitted by at least one first radiation source (6) has a different wavelength than radiation emitted by at least one second radiation source (22).

5. The centrifuge (1) according to any one of claims 1 to 4, **characterized in that** at least two radiation sources are arranged mirror-symmetrically to one another with respect to a mirror plane (S) which contains a center axis of the rotor shaft (14).

6. The centrifuge (1) according to any one of claims 1 to 5, **characterized in that** the centrifuge (1) has at least one reflection body which is introduced into the receiving section (7) of the rotor (4), wherein the reflection body has at least one surface for reflecting radiation.

7. The centrifuge (1) according to any one of claims 1 to 6, **characterized in that**
a. the rotor (4) has a reflection surface which is oriented in such a way that it reflects the radiation emitted by the radiation source (6, 22) into a predetermined housing region and/or **in that**
b. a part of the inner housing surface (11) is oriented in such a way that it reflects the radiation emitted by the radiation source (6, 22) into a predetermined rotor housing region and/or a predetermined rotor region.

8. The centrifuge (1) according to any one of claims 1 to 7, **characterized in that** the centrifuge (1) has at least one reflection body (36), wherein
a. the reflection body (36) is connected to the rotor (5) in a non-rotatable manner and/or **in that**
b. the reflection body (36) is attached to a rotor side (38) which is arranged offset in the tangential direction with respect to the receiving section (7) and/or **in that**
c. the reflection body (36) has a reflection surface (37) which extends axially and/or runs transversely to the center axis of the rotor shaft (14) and/or **in that**
d. the centrifuge (1) has at least two reflection bodies (36) which are arranged on opposite rotor sides (38).

9. The centrifuge (1) according to any one of claims 1 to 8, **characterized in that** the at least one radiation source (6, 22) is connected to the rotor (5) in a non-rotatable manner.

10. The centrifuge (1) according to claim 9, **characterized in that**
a. the radiation source (6, 22) is oriented such that the radiation from the rotor (5) is emitted radially outwards and/or **in that**
b. the centrifuge (1) has an electrical line for the connection of the radiation source (6, 22) to an energy source, wherein the electrical line runs at least partially through the rotor shaft (14).

11. The centrifuge (1) according to any one of claims 1 to 10, **characterized in that**
a. the emitter (31) is attached to a metallic conducting element (33) of the heat transfer section (39) and/or **in that**
b. the heat transfer section (39) has a conductive paste (34), by means of which a metallic conducting element (33) of the heat transfer section (39) is connected to the rotor housing (8) in a thermally conductive manner and/or **in that**
c. the radiation source (6, 22) has a printed circuit board (35) which is directly connected to the metallic conducting element (33).

12. The centrifuge (1) according to any one of claims 1 to 11, **characterized in that** the centrifuge (1) has a control device (18), which effects the following:
a. during a washing operation, the radiation source (6, 22) does not emit any radiation and/or
b. the radiation source (6, 22) emits radiation when no sample carrier (2) is arranged in the rotor (5) and/or
c. the rotor (5) is brought into a predetermined position in a decontamination operation, which differs from a position in which the sample carrier (2) can be inserted into the rotor (5) or ejected from the rotor (5) and/or
d. the rotor (5) is rotated during the decontamination operation.

13. A method for operating a centrifuge (1) according to any one of the preceding claims 1 to 12, wherein a sample carrier (2), which has at least one container (3) for receiving a liquid sample (5), is rotated by means of a rotor (5) in a rotor chamber (4), **characterized in that** radiation with a wavelength of at most 350 nm is emitted into the rotor chamber (4).

14. The method according to claim 13, **characterized in that**
a. the radiation is emitted temporally before or after a washing operation and/or **in that**
b. the radiation is emitted when the rotor (5) does not receive a sample carrier (2) and/or **in that**
c. the radiation is emitted at predetermined times and/or for a predetermined period of time.

15. The method according to claim 13 or 14, **characterized in that**
a. the rotor (5) is rotated during the decontamination operation and/or **in that**
b. a rotor speed during the washing operation is greater than during a decontamination operation in which radiation is emitted and/or **in that**
c. the rotor (5) is rotated in a decontamination operation, into a position which differs from a position in which the sample carrier (2) is introduced into the rotor (5) or removed from the rotor (5) and/or **in that**
d. the radiation source (6, 22) is switched off as soon as the flap (19) does not cover the passage (16).

## Revendications

1. Centrifugeuse (1) pour faire tourner un porte-échantillon (2), lequel comprend au moins un récipient (3) pour recevoir un échantillon liquide, comportant un rotor (5) rotatif, lequel comporte au moins une section de réception (7) pour recevoir le porte-échantillon (2), et un espace de rotor (4), dans lequel est disposé le rotor (5), la centrifugeuse (1) comportant au moins une source de rayonnement (6, 22) pour émettre un rayonnement dans l'espace de rotor (4) à une longueur d'onde de 350 nm au maximum, dans laquelle l'espace de rotor (4) est délimité par un carter de rotor (8), **caractérisée en ce que** la source de rayonnement (6, 22) comporte un émetteur de rayonnement (31), lequel est relié par thermoconductivité au moyen d'une section de transmission de chaleur (39), au carter de rotor (8).

2. Centrifugeuse (1) selon la revendication 1, **caractérisée en ce que**
a. l'au moins une source de rayonnement (6, 22) est disposée sur ou dans le carter de rotor (8) et/ou **en ce que**
b. une paroi (12) avant et/ou une paroi (13) arrière du carter de rotor (8) comporte un évidement dans lequel est disposée l'au moins une source de rayonnement (6, 22) et/ou
c. **en ce qu'**une coque supérieure et/ou une coque inférieure (9, 10) du carter de rotor (8) comporte un évidement dans lequel est disposée l'au moins une source de rayonnement (6, 22).

3. Centrifugeuse (1) selon la revendication 1 ou 2, **caractérisée en ce que**
a. la centrifugeuse (1) comporte un couvercle (21), lequel sépare fluidiquement la source de rayonnement (6, 22) de l'espace de rotor (4) et/ou lequel est conçu de telle sorte qu'il transmet le rayonnement de la source de rayonnement (6, 22) et/ou **en ce que**
b. la centrifugeuse (1) comporte au moins une conduite électrique pour relier de manière électrique la source de rayonnement (6, 22) à une source d'énergie, dans laquelle la conduite électrique s'étend au moins à certains endroits à travers la paroi (12) avant et/ou la paroi (13) arrière et/ou que
c. la source de rayonnement (6, 22) est orientée de telle sorte que le rayonnement frappe le rotor (5) et/ou une surface intérieure de carter (11).

4. Centrifugeuse (1) selon la revendication 2 ou 3, **caractérisée en ce qu'**elle comporte plusieurs sources de rayonnement (6, 22), dans laquelle un rayonnement émis par au moins une première source de rayonnement (6) présente une longueur d'onde différente qu'un rayonnement émis par au moins une seconde source de rayonnement (22).

5. Centrifugeuse (1) selon l'une quelconque des revendications 1 à 4, **caractérisée en ce que** au moins deux sources de rayonnement sont disposées de manière symétrique l'une par rapport à l'autre par rapport à un plan du miroir (S), lequel comporte un axe médian d'un arbre de rotor (14).

6. Centrifugeuse (1) selon l'une quelconque des revendications 1 à 5, **caractérisée en ce que** la centrifugeuse (1) comporte au moins un corps réfléchissant, lequel est monté dans la section de réception (7) du rotor (4), le corps réfléchissant comportant au moins une surface servant à réfléchir le rayonnement.

7. Centrifugeuse (1) selon l'une quelconque des revendications 1 à 6, **caractérisée en ce que**
a. le rotor (4) comporte une surface réfléchissante, laquelle est orientée de telle sorte qu'elle réfléchit le rayonnement émis par la source de rayonnement (6, 22) dans une zone de carter prédéterminée et/ou
b. **en ce qu'**une partie de la surface intérieure du carter (11) est orientée de telle sorte qu'elle réfléchit le rayonnement émis par la source de rayonnement (6, 22) dans une zone prédéterminée du carter du rotor et/ou une zone prédéterminée du rotor.

8. Centrifugeuse (1) selon l'une quelconque des revendications 1 à 7, **caractérisée en ce que** la centrifugeuse (1) comporte au moins un corps réfléchissant (36),
a. le corps réfléchissant (36) étant solidaire en rotation avec le rotor (5) et/ou **en ce que**
b. le corps réfléchissant (36) est monté sur un côté de rotor (38), lequel est décalé dans la direction tangentielle par rapport à la section de réception (7) et/ou **en ce que**
c. le corps réfléchissant (36) comporte la surface réfléchissante (37), laquelle s'étend axialement et/ou s'étend transversalement à l'axe médian de l'arbre de rotor (14) et/ou **en ce que**
d. la centrifugeuse (1) comporte au moins deux corps réfléchissants (36), lesquels sont disposés sur des côtés de rotor (38) se faisant face.

9. Centrifugeuse (1) selon l'une quelconque des revendications 1 à 8, **caractérisée en ce que** l'au moins une source de rayonnement (6, 22) est solidaire en rotation avec le rotor (5).

10. Centrifugeuse (1) selon la revendication 9, **caractérisée en ce que**
a. la source de rayonnement (6, 22) est orientée de telle sorte que le rayonnement est émis radialement vers l'extérieur par le rotor (5) et/ou **en ce que**
b. la centrifugeuse (1) comprend une conduite électrique pour connecter la source de rayonnement (6, 22) à une source d'énergie, la ligne électrique s'étendant au moins à certains endroits à travers l'arbre de rotor (14).

11. Centrifugeuse (1) selon l'une quelconque des revendications 1 à 10, **caractérisée en ce que**
a. l'émetteur de rayonnement (31) est monté sur un élément conducteur (33) métallique de la section de transfert de chaleur (39) et/ou **en ce que**
b. la section de transfert de chaleur (39) comporte une pâte conductrice (34) au moyen de laquelle un élément conducteur (33) métallique de la section de transfert de chaleur (39) est relié par thermodconductivité au carter de rotor (8) et/ou **en ce que**
c. la source de rayonnement (6, 22) comprend une carte de circuit imprimé (35), laquelle est directement connectée à l'élément conducteur (33) métallique.

12. Centrifugeuse (1) selon l'une quelconque des revendications 1 à 11, **caractérisée en ce que** la centrifugeuse (1) comprend un dispositif de commande (18), lequel fait en sorte que
a. lors d'une opération de lavage, la source de rayonnement (6, 22) n'émet aucun rayonnement et/ou **en ce que**
b. la source de rayonnement (6, 22) émet le rayonnement lorsqu'aucun porte-échantillon (2) n'est disposé dans le rotor (5) et/ou **en ce que**
c. le rotor (5) est amené, dans un mode de décontamination, dans une position prédéterminée, laquelle diffère d'une position dans laquelle le porte-échantillon (2) peut être monté dans le rotor (5) ou démonté du rotor (5) et/ou **en ce que**
d. le rotor (5) tourne pendant le mode de décontamination.

13. Procédé de fonctionnement d'une centrifugeuse (1) selon l'une quelconque des revendications précédentes 1 à 12, dans lequel un porte-échantillon (2), lequel comporte au moins un récipient (3) pour recevoir un échantillon (5) liquide, est mis en rotation, au moyen d'un rotor (5), dans un espace de rotor (4), **caractérisé en ce qu'**un rayonnement d'une longueur d'onde d'au plus 350 nm est émis dans l'espace de rotor (4).

14. Procédé selon la revendication 13, **caractérisé en ce que**
a. le rayonnement est émis temporellement avant ou après un mode de lavage et/ou **en ce que**
b. le rayonnement est émis lorsque le rotor (5) ne reçoit pas de porte-échantillon (2) et/ou **en ce que**
c. le rayonnement est émis à des moments prédéfinis et/ou pendant une durée prédéfinie.

15. Procédé selon la revendication 13 ou 14, **caractérisé en ce que**
a. le rotor (5) est mis en rotation en mode de décontamination et/ou **en ce que**
b. une vitesse de rotation du rotor en mode de lavage est supérieure à celle en mode de décontamination lors de laquelle un rayonnement est émis et/ou **en ce que**
c. le rotor (5) est mis en rotation, en mode de décontamination, dans une position, laquelle diffère de la position dans laquelle le porte-échantillon (2) est monté dans le rotor (5) ou démonté du rotor (5) et/ou **en ce que**
d. la source de rayonnement (6, 22) est mise hors tension dès qu'un volet (19) n'obstrue pas le passage (16).
